Europäisches Patentamt

European Patent Office

Office européen des brevets

(11) Publication number: **0 422 290 A1**

# EUROPEAN PATENT APPLICATION

(21) Application number: 89310294.7

(22) Date of filing: 09.10.89

(51) Int. Cl.⁵: **A61K 9/00**, A61K 9/10, A61K 47/02, A61K 47/12

(43) Date of publication of application:
**17.04.91 Bulletin 91/16**

(84) Designated Contracting States:
**AT BE CH DE FR GB IT LI LU NL SE**

(71) Applicant: **ELI LILLY AND COMPANY**
**Lilly Corporate Center**
**Indianapolis Indiana 46285(US)**

(72) Inventor: **Cuff, George William**
**2126 East 61st Place**
**Indianapolis Indiana 46220(US)**

(74) Representative: **Tapping, Kenneth George et al**
**Erl Wood Manor**
**Windlesham Surrey, GU20 6PH(GB)**

(54) **Novel drug delivery system.**

(57) A unique pharmaceutical delivery system comprising a physiologically acceptable nonaqueous liquid such as an edible oil and a thickening agent such as colloidal silicon dioxide which together form a semi-solid having the consistency of a pudding is provided. This novel system masks unacceptable taste, does not adversely affect the stability of the drug, and accommodates large doses. Its primary advantage is its ease of administration to very young or debilitated patients.

EP 0 422 290 A1

## NOVEL DRUG DELIVERY SYSTEM

Pharmaceutical drug delivery systems are designed to deliver an effective amount of a therapeutic drug to the site of action. Many problems confront the designer of efficient drug delivery systems. One such problem relates to the effective delivery of orally administered drugs when the patient is an infant, a small child or is debilitated. To solve this problem, liquid dosage forms have generally been used. These liquid forms, however, often have a disagreeable taste. Parents frequently encounter great difficulty in administering them to resistant children. Even when resistance is not a problem, it is difficult to administer them without losing medication via spillage. Similarly, in the debilitated, a shaky hand frequently makes it hard to measure and self-administer a liquid preparation. To solve this dilemma, some parents and patients mix the liquid formulation with food. This presents problems, however, in knowing how much medicine is consumed and in not knowing whether the medicine interacts with food constituents.

Another problem confronting the designer of efficient drug delivery systems occurs when the amount of drug which must be administered is fairly large. Presenting the drug in a solid dosage form is troublesome, because the dosage form must be easily swallowed. To avoid this problem, such drugs are often prepared as chewable tablets or liquids. Masking the taste of the drug in a chewable tablet is sometimes impossible, and the amount of liquid which must be consumed can also be sizable. Thus, there continues to be a need for effective drug delivery systems which are easy to administer and acceptable to very young and debilitated patients.

This invention provides a novel pharmaceutical vehicle which is useful for delivering drugs orally to infants, children and debilitated patients. The drug delivery system consists of a clear, physiologically acceptable nonaqueous liquid and a thickening agent, such as colloidal silicon dioxide, which together form a semi-solid or gel having the consistency of a pudding. Some of the advantages of this system are: 1) it provides a high level of patient acceptability; 2) it is easy to administer; 3) it masks the taste of drugs having an unacceptable taste; 4) it permits the administration of large doses in a consumable volume ; 5) it provides for the administration of varying dosages; and 6) it does not adversely affect the stability of the drug being administered.

The pharmaceutical composition or drug delivery system of this invention is a semi-solid or gel having the consistency of a pudding. This delivery system provides the drug in a vehicle which is readily acceptable to the patient. Furthermore, this system provides a vehicle in which the amount of drug administered may be adjusted by merely increasing or decreasing the volume given, as in liquid formulations. The loss of drug through spills is minimized because of the semi-solid nature of the system.

A special advantage of this drug delivery system is its ability to mask unpleasant taste. Another special advantage is that it permits large doses to be administered in a small volume. Furthermore, this system does not adversely affect the stability of the drug being administered.

Yet another advantage of this system is that the semi-solid state is attained without the presence of water. Thus, drugs which would be adversely affected in a liquid formulation by the presence of water can be safely administered in this semi-solid system.

The drug delivery system of this invention consists of a physiologically acceptable nonaqueous liquid and a thickening agent or agents which together form a semi-solid having the consistency of a pudding.

Physiologically acceptable nonaqueous liquids which are especially useful in this system are edible oils. Examples of such oils are fractionated coconut oil, sesame oil, rapeseed oil, soybean oil, peanut oil and linoleic acid.

A variety of thickening agents may be used in this system. Examples of useful thickening agents are colloidal silicon dioxide, aluminum stearate, high molecular weight polyethyleneglycols, moderate to high melting point waxes or fats, hydrophobic colloidal silicon dioxides and other fumed or colloidal metal oxides such as aluminum oxide. Colloidal silicon dioxide is a particularly suitable thickening agent.

The thickening agent is added in an amount sufficient to give the delivery system the consistency of a pudding. The viscosity range of such pudding is in the range of from about 5,000 to about 100,000 centipoise at room temperature. It may be desirable to refrigerate the delivery system to protect the stability of the drug. If refrigerated, the pudding viscosity may increase slightly.

In general, the drug delivery system of the present invention will contain from about 0.01 to about 35% (by weight) of the drug, from about 1 to about 20% (by weight) of the thickening agent and from about 45 to about 99% (by weight) of the liquid medium. Preferably, the delivery system will contain from about 0.1 to about 10% of the drug, from about 3 to about 6% of the thickening agent and from about 75 to about 90% of the liquid medium.

In addition to the drug, the drug delivery vehicle may also contain buffering agents and preservatives.

Suitable preservatives which may be used in the drug delivery system are sodium benzoate, sorbic acid and the methyl and propyl ester of p-hydroxybenzoic acid. These agents may be present in amounts of from about 0.001 to about 5% by weight, but are preferably present in amounts of from about 0.01 to about 2%.

Flavoring agents can also be included in the drug delivery vehicle to provide greater patient acceptance. Examples of suitable flavoring agents include cherry flavor, orange-lemon flavor, strawberry flavor, chocolate, peppermint oil, double distilled eucalyptol, anethol, methyl salicylate, oil cassia, cinnamic aldehyde, and the like. Flavoring agents are generally present in amounts of from about 0.01 to about 5%, preferably from about 0.1% to about 1%.

In addition to the flavoring agents, sweetening agents may also be added to provide an acceptable taste. Suitable sweetening agents include sucrose, fructose, glucose, sodium saccharin, aspartame and sodium cyclamate. Mixtures of these agents can also be used. The amount of sweetening agent in the vehicle will vary, depending on the equivalent sweetening power of the agent.

The drug delivery system of this invention may be used to deliver any pharmaceutically active material. Drugs which are insoluble in the nonaqueous liquid medium are particularly suitable for this system. Because such drugs are insoluble in the system, the stability of the drug is assured. Drugs which are insoluble in the medium and also have a distasteful taste are especially good candidates for this system because the taste is more readily masked. Drugs which are soluble in the nonaqueous liquid medium may also be used, provided the taste is acceptable and the stability can be preserved. In those case in which the distasteful taste is not sufficiently masked, then coating the drug with an acceptable material may be necessary to augment the taste-masking effect of this sytem.

Examples of suitable drugs are antacids, antiflatulents, antibacterials, analgesics, antihistamines and decongestants (cold preparations), antitussives (cough preparations), anti-inflammatories, antivirals, antifungals, antidiarrheals, laxatives and anti-asthma drugs.

This system is particularly advantageous for antibacterial substances. Examples of such substances are the $\beta$-lactam antibiotics, such as cephalexin, cephradine, cefaclor, amoxacillin, penicillin V, cefoxitin, cefuroxime axetil and cefadroxil. Other antibacterial agents include macrolides such as erythromycin, tetracyclines, chloramphenicol, neomycin, aminoglycoside antibiotics such as kanamycin and nalidixic acids and analogs such as norfloxacin. Antimicrobial combination drugs may also be used.

Typical antacids are aluminum hydroxide, magnesium hydroxide and sodium bicarbonate. Simethicone is an exemplary antiflatulent.

Examples of antihistamines and decongestants are pyrilamine, cholpheniramine, tetrahydrazoline, phenylpropanolamine and antazoline.

A typical antitussive is dextromethorphan.

Anti-inflammatory agents include cortisone, hydrocortisone, $\beta$-methasone, dexamethasone, fluocortolone, prednisolone, triamcinolone, indomethacin, sulindac, and the like.

Antiviral agents include acylovir and interferon.

Antifungal agents include griseofulvin and nystatin.

Useful antidiarrheals include kaolin, pectin, bismuth subsalicylate, paregoric, loperamide and diphenoxylate.

Laxatives which may be incorporated include mineral oil, danthron and bisacodyl.

Examples of anti-asthma drugs are theopylline, ephedrine, beclomethasone dipropionate and epinephrine.

Typically, the drug delivery system of this invention contains from about 0.001 to about 40% of the drug on a weight-to-weight basis. Thus, one gram of the semi-solid system would provide about 0.1 mg to about 400 mg of drug. The amount and nature of the drug used will, of course, depend upon the requirements of the patient. Thus, for example, from about 2.5 to about 10% of cephalexin in the drug delivery system would provide an effective amount of the drug when administered. The amount of vehicle administered depends upon a number of factors, such as the weight and condition of the patient.

Generally, the first step in preparing the drug delivery system is to mix some of the liquid carrier, the preservatives and the sweetening agents. This mixture is heated to remove any moisture that may be present and to assure uniform dispersion of any waxlike components such as aluminum stearate, since these melt and are easier to disperse as a melt.

The next step is to add the flavoring agents and dyes. Because they are possibly unstable when heated, these are added after the first ingredients have been allowed to cool.

The thickening agent is added last because it is easier to get a uniform dispersion of the other components before the product is thickened. In addition, excessive shearing after the thickening agent is added causes a loss of viscosity in the product.

3

The drug delivery system of this invention can be packaged in a variety of ways. A wide-mouthed jar, a squeezable tube, and individually packaged doses are examples of convenient packaging methods.

The following examples illustrate the preparation of the drug delivery system of this invention.

Example 1

| Cephalexin Gel (500 mg/5 mL) | | |
|---|---|---|
| 1. To a suitable container add: | | |
| | | Amount |
| | Fractionated Coconut Oil | 600 mL |
| 2. Heat to 90-100° C. and maintain temperature 3. Add while mixing: | | |
| | Lecithin, Refined Grade | 7 g |
| | Butylparaben | 0.4 g |
| 4. Mix until dissolved and add | | |
| | Castor Oil, Hydrogenated | 3.5 g |
| | Aluminum Stearate | 10 g |
| 5. Mix until completely dispersed 6. Add slowly: | | |
| | Sucrose (granulated cane, ≦ 100 mesh) | 300 g |
| | Sodium Chloride, Powdered | 0.5 g |
| 7. Mix while maintaining temperature at 90-100° for 3 hours 8. Turn off heat and add: | | |
| | Fractionated Coconut Oil | 300 mL |
| 9. Mix continuously until cooled to room temperature 10. Add: | | |
| | D&C Yellow No. 10 Aluminum Lake | 150 mg |
| | Imitation Guarana Liquid | 10 mL |
| | Vanillin | 1 g |
| 11. Mix thoroughly 12. Add slowly: | | |
| | Cephalexin for Suspension | 111.7 g |
| 13. Add and mix thoroughly: | | |
| | Colloidal Silicon Dioxide | 25 g |
| 14. Add fractionated coconut oil to make one liter and mix thoroughly. | | |

This gel delivery system contains 2.5% colloidal silicon dioxide and provides 500 ml of cephalexin per mL of product.

Example 2

Cephalexin Gel (500 mg/5 mL)

The procedure of Example 1 is used, but the amount of colloidal silicon dioxide is increased to 4%, giving a firmer semi-solid which tastes very good.

## Example 3

| Cephalexin Gel (250 mg/5 mL) | | |
|---|---|---|
| 1. Following in general the procedure of Example 1, prepare a vehicle using the following ingredients: | | |
| | Vehicle | |
| | Ingredient | Amount |
| | Fractionated Coconut Oil | q.s. to 1 liter |
| | Lecithin, Refined Grade | 7 g |
| | Butylparaben | 400 mg |
| | Aluminum Stearate | 10 g |
| | Castor Oil, Hydrogenated | 3.5 g |
| | Sucrose, Powdered | 300 g |
| | Sodium Chloride, Powdered | 500 mg |
| 2. Use 1.03 kg of the above vehicle per liter of final product and add the following: | | |
| | D&C Yellow No. 10 Aluminum Lake | 750 mg |
| | Imitation Guarana Liquid | 5 mL |
| 3. | Cephalexin for Suspension | 54.2 g |
| 4. Add to this: | | |
| | Colloidal Silicon Dioxide | 40 g |

## Example 4

Follow the procedure of Example 3, but substitute for steps 2 and 3:

| 2. Add: | | |
|---|---|---|
| | Ingredient | Amount |
| | D&C Red No. 6 Barium Lake | 200 mg |
| | Imitation Guarana Liquid | 5 mL |
| 3. | Cephalexin for Suspension | 27.1 g |

## Example 5

Cefaclor Gel (250 mg/5 mL)

Follow the procedure of Example 3, but substitute for steps 2 and 3:

| 2. Add: | | |
|---|---|---|
| | Ingredient | Amount |
| | D&C Yellow No. 10 Aluminum Lake | 450 mg |
| | Imitation Guarana Liquid | 625 mg |
| | Imitation Strawberry | 1.500 g |
| | Vanillin | 500 mg |
| 3. | Cefaclor Monohydrate | 54.744 g |
| (Based on 959 mcg/mL potency--includes 5% excess) | | |

## Example 6

Cefaclor Gel (125 mg/5 mL)

Follow the procedure for Example 3, but substitute for steps 2 and 3:

| 2. Add: | | |
|---|---|---|
| | FD&C Red No. 3 HT Aluminum Lake | 300 mg |
| | Vanillin | 500 mg |
| | Imitation Strawberry | 1.875 g |
| 3. | Cefaclor Monohydrate | 27.372 g |

## Example 7

Antacid Gel (420 mg/5 mL)

Follow the procedure for Example 3, but substitute for step 3:

| | Ingredient | Amount |
|---|---|---|
| 3. | Aluminum Hydroxide (dried gel, USP) | 40 g |
| | Magnesium Hydroxide | 40 g |
| | Simethicone | 4 g |

## Example 8

Antitussive--Decongestant Gel

Follow the procedure for Example 3, but use peanut oil instead of coconut oil in step 1 and substitute for step 3:

|   | Ingredient | Amount |
|---|---|---|
| 3. | Dextromethorphan Hydrobromide | 2 g |
|   | Pseudoephedrine Hydrochloride | 6 g |
|   | Guaifenesin | 20 g |

Example 9

Antidiarrheal Gel

Follow the procedure for Example 3, but use soybean oil instead of coconut oil in step 1 and substitute for step 3:

|   | Ingredient | Amount |
|---|---|---|
| 3. | Kaolin | 200 g |
|   | Pectin | 4.3 g |

**Claims**

1. A semi-solid pharmaceutical composition for oral administration comprising:
a) an orally effective amount of drug selected from the group consisting of antacids, antiflatulents, antibacterials, analgesics, antihistamines and decongestants, antitussives, antiinflammatories, antivirals, antifungals, antidiarrheals, laxatives and anti-asthma drugs;
b) a physiologically acceptable nonaqueous liquid; and
c) from about 1 to about 20% by weight of a thickening agent which, together with the liquid, forms a semi-solid having the consistency of pudding.

2. A composition of claim 1 wherein the anti-bacterial substances are selected from the group consisting of $\beta$-lactam antibiotics, tetracyclines, chloramphenicol, neomycin, aminoglycoside antibiotics and nalidixic acid and its analogs.

3. A composition of claim 2 wherein the $\beta$-lactam antibiotic is selected from cephalexin, cephradine, cefaclor, amoxacillin, penicillin V, cefoxitin, cefuroxime axetil and cefadroxil.

4. A composition of claim 1 wherein the analgesic is selected from aspirin, acetaminophen, propoxyphene, ibuprofen and pentazocine.

5. A composition of claim 1 wherein the antacid is selected from aluminum hydroxide, magnesium hydroxide and sodium bicarbonate.

6. A composition of claim 1 wherein the antihistamine or decongestant is selected from pyrilamine, cholpheniramine, tetrahydrozaline, phenylpropanolamine or antazolene.

7. A composition of claim 1 wherein the anti-inflammatory is selected from cortisone, hydrocortisone, $\beta$-methasone, dexamethasone, fluocortolone, prednisolone, triamcinolone, indomethacin, sulindac, and the like.

8. A composition of claim 1 wherein the thickening agent is selected from colloidal silicon dioxide, fumed or colloidal metal oxides, aluminum stearate, high molecular weight polyethylene glycols and moderate to high melting point waxes or fats.

9. A composition of claim 1 which contains about 3 to about 6% by weight of the thickening agent.

10. A composition of claim 1 wherein the composition has a viscosity in the range of about 5,000 to about 100,000 centipoise at room temperature.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 784 851 (G.L. ROHWER et al.) * whole article * | 1,5,8 | A 61 K 9/00 A 61 K 9/10 A 61 K 47/02 A 61 K 47/12 |
| X | US-A-4 145 429 (C.D. CLARKE et al.) * column 1, lines 23-28; claims * | 1-3,9 | |
| X | US-A-4 079 138 (SONG-LING et al.) * column 2, lines 29-50; column 3, lines 10-39; column 4, lines 6-32; claims * | 1-4,8,9 | |
| X | EP-A-0 225 189 (R.P. SCHERER CORPORATION) * examples 1-9; claims * | 1-3,8,9 | |
| X | US-A-2 951 014 (H.G. GARMAN et al.) * column 2, lines 14-30,63-70; examples; claims * | 1,2,4,8 | |
| X | EP-A-0 310 801 (ABBOTT LABORATORIES) * page 2, lines 35-40; page 3, lines 2-15; claims * | 1,8,9 | TECHNICAL FIELDS SEARCHED (Int. Cl.5) |
| A | EP-A-0 314 421 (LILLY INDUSTRIES LTD) * claims * | 1-10 | A 61 K |

The present search report has been drawn up for all claims

| Place of search | Date of completion of the search | Examiner |
|---|---|---|
| BERLIN | 12-06-1990 | SIATOU E |

EPO FORM 1503 03.82 (P0401)